# FASCICULE DE BREVET EUROPEEN

(11) **EP 1 100 286 B1**
(45) Date de publication et mention de la délivrance du brevet: **27.08.2003**
(21) Numéro de dépôt: 00460056.5
(22) Date de dépôt: 25.10.2000
(51) Int. Cl.: H04R 1/46, A61F 11/04, H04R 25/00

(54) **Dispositif de stimulation tactile destiné à être utilisé par une personne sourde**
Vorrichtung zur taktilen Stimulation für Taube
Tactile stimulation device for deaf

(30) Priorité: 10.11.1999 FR 9914470
(43) Date de publication de la demande: 16.05.2001
(73) Titulaire: Coudon, Jean-Max, 49240 Avrille (FR)
(72) Inventeur: Coudon, Jean-Max, 49240 Avrille (FR)
(74) Mandataire: Maillet, Alain

(56) Documents cités:
- EP-A- 0 184 332
- DE-A- 3 322 108
- DE-C- 3 834 442
- FR-A- 2 577 739
- US-A- 4 250 637
- US-A- 5 035 242

## Description

La présente invention concerne un dispositif de stimulation tactile apte à transformer un signal audio en vibrations pouvant être perçues par la peau d'une personne.

Un tel dispositif comportant un transducteur électroacoustique est, par exemple, connu du document US-A-4 250 637.

Le domaine de l'invention intéresse particulièrement les personnes sourdes ou les personnes présentant une déficience auditive dont l'appareil auditif-est déficient ou non fonctionnel.

Pour qu'une personne sourde puisse comprendre un interlocuteur, la méthode la plus connue consiste en ce que cette personne apprenne à lire sur les lèvres.

Il est cependant des situations où cette personne ne peut apercevoir son interlocuteur, par exemple lors du fonctionnement d'un interphone ou bien lorsque la personne tente de répondre à la fin d'un message de son interlocuteur, transmis par un interphone.

Il existe également un réel besoin de renforcer la capacité de communication entre une personne sourde et un interlocuteur lors de la lecture labiale en l'aidant à percevoir la différenciation vibratoire des différents phonèmes et aussi en lui permettant d'ajuster sa voix en fonction du bruit ambiant.

On a tenté de résoudre ce problème en proposant des dispositifs de stimulation tactile comportant en général un microphone raccordé à un boîtier incluant une source de courant électrique ainsi qu'une carte électronique destinée à convertir le signal électrique fourni par le microphone en un signal électrique destiné à mettre en oeuvre un transducteur électroacoustique.

Un transducteur électroacoustique est raccordé au boîtier, et est maintenu sur la peau de la personne, par exemple par un bracelet mettant en contact le transducteur avec le poignet de la personne désirant l'utiliser.

Après l'acquisition d'une technique de reconnaissance des vibrations transmises par le transducteur électroacoustique sur la peau de la personne, celle-ci est capable d'interpréter les vibrations reçues par la peau et transmises au cerveau par les terminaisons nerveuses de la peau, de manière à comprendre la parole de son interlocuteur.

Cependant, ce type de dispositif ne donne pas entière satisfaction car il est encombrant, nécessite des raccordements électriques entre ses différents constituants et impose le port permanent d'un bracelet ou d'un dispositif de fixation du transducteur sur la peau.

Par ailleurs, la peau humaine ne peut percevoir des vibrations dont la fréquence est supérieure à 1000 Hz.

Alors que la voix humaine produit des sons dont les fréquences les plus importantes pour son intelligibilité sont comprises dans la bande de fréquence de 1000 à 3000 Hz, il en va de même pour la bande passante des interphones et téléphones qui s'étend jusqu'à 3000 Hz.

Aussi, il est apparu intéressant de pouvoir exploiter les informations de la voix humaine dans la bande de fréquence de 1000 à 3000 Hz pour les exploiter dans le dispositif de l'invention.

Le premier but de l'invention est donc de proposer un dispositif de stimulation tactile d'une construction compacte et extrêmement simple à mettre en oeuvre.

Un autre but de l'invention est de proposer un dispositif de stimulation tactile qui exploite les informations transmises dans la voix humaine, dont la fréquence est comprise entre 1000 et 3000 Hz.

A cet effet, le dispositif de l'invention comporte notamment un transducteur électroacoustique et est caractérisé en ce qu'il est constitué d'un boîtier préhensible dans une main et comportant un senseur de mise sous tension associé au transducteur électroacoustique, et destiné à permettre la mise sous tension du dispositif lorsque l'on touche ledit senseur.

Ainsi; le dispositif de l'invention est compact et facile à mettre en oeuvre.

Selon une autre caractéristique de l'invention, le senseur est constitué d'un support isolant pourvu de bandes conductrices isolées électriquement les unes des autres et raccordées à l'entrée d'un détecteur de contact.

Selon une autre caractéristique de l'invention, les bandes conductrices ont chacune la forme d'une spirale et sont imbriquées les unes dans les autres.

Selon une autre caractéristique de l'invention, le senseur est collé sur la membrane du transducteur électroacoustique.

Selon une autre caractéristique de l'invention, le dispositif comporte un filtre fréquentiel passe-bas qui sélectionne les fréquences inférieures à 800 Hz.

Selon une autre caractéristique de l'invention, le dispositif comporte un filtre fréquentiel passe-bande raccordé à un générateur de fréquence apte à moduler en amplitude sur une fréquence déterminée le signal reçu dudit filtre fréquentiel.

Selon une autre caractéristique de l'invention, le filtre fréquentiel passe-bande sélectionne la plage de fréquences comprise entre 1000 Hz et 4000 Hz.

Selon une autre caractéristique de l'invention, la fréquence de fonctionnement du générateur de fréquence est de 900 Hz.

Ainsi, les fréquences aiguës de la voix sont transcrites par le dispositif de l'invention.

Selon une autre caractéristique de l'invention, le bouton de mise sous tension du dispositif peut prendre une position d'arrêt destinée à couper l'alimentation électrique du dispositif, une position de mise sous tension du dispositif et une position de veille pouvant mettre en marche le dispositif par l'intermédiaire du détecteur de contact.

Selon une autre caractéristique de l'invention, le dispositif comporte un connecteur d'entrée prévu pour recevoir un microphone amovible ou un accessoire de réception T comme un récepteur à boucles magnétiques.

Selon une autre caractéristique de l'invention, le dispositif comporte un connecteur de sortie prévu pour recevoir un transducteur électroacoustique extérieur.

Selon une autre caractéristique de l'invention, le dispositif comporte une molette de réglage permettant d'agir sur un potentiomètre afin de moduler l'intensité du courant électrique destiné à alimenter le transducteur électroacoustique.

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels:
la Fig. 1 représente une vue de face d'un dispositif de stimulation tactile selon l'invention,
la Fig. 2 représente une vue en coupe d'un transducteur électroacoustique selon l'invention,
la Fig. 3 représente une vue de dessus d'un transducteur électroacoustique selon l'invention et,
la Fig. 4 représente une vue schématique du fonctionnement d'un dispositif de stimulation tactile selon l'invention.

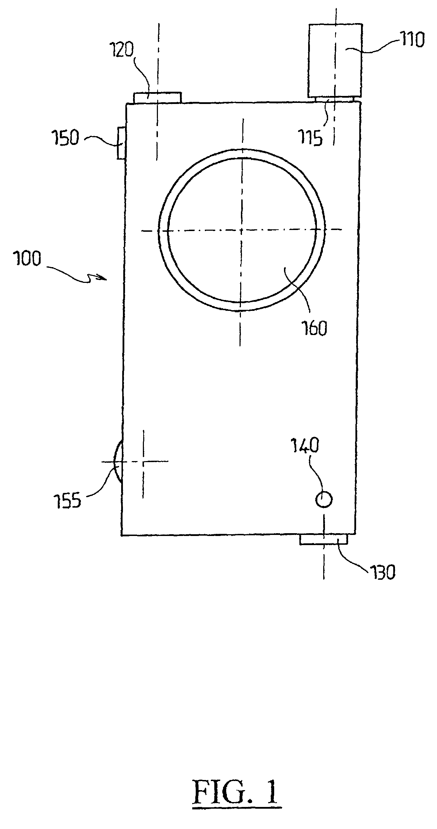

Le dispositif de stimulation tactile représenté à la Fig. 1 comporte un boîtier 100 parallélépipédique incorporant un connecteur électrique d'entrée 115 dans lequel est monté un microphone amovible 110.

Le connecteur électrique d'entrée 115 peut également être utilisé pour recevoir un accessoire de réception comme un récepteur à boucles magnétiques en remplacement du microphone amovible 110.

Un connecteur électrique de sortie 120 est prévu dans le boîtier 100 pour permettre le raccordement d'un autre transducteur électroacoustique extérieur de type "intégral", c'est-à-dire qui permet de saisir les vibrations dans tous les doigts qui le palpent aussi bien au recto qu'au verso dudit transducteur.

Un connecteur d'alimentation électrique 130 est installé dans le boîtier 100. Il permet une alimentation électrique extérieure du dispositif dont le fonctionnement est mis en évidence par un témoin visuel 140 d'alimentation extérieure.

Le boîtier 100 du dispositif de stimulation incorpore aussi un bouton de mise sous tension 150 dudit dispositif pouvant prendre trois positions : une position d'arrêt pour couper l'alimentation électrique du dispositif, une position de mise sous tension du dispositif et une position de veille pouvant mettre en marche le dispositif lorsqu'il est sollicité comme cela est expliqué dans la suite de la description.

Un transducteur électroacoustique 160 est monté sur l'une des faces principales du boîtier 100.

Une molette de réglage 155 permet d'agir sur un potentiomètre permettant de moduler l'intensité du courant électrique destiné à alimenter le transducteur électroacoustique 160.

A l'intérieur du boîtier 100 est prévue notamment une source d'alimentation électrique comme des piles ou des accus rechargeables. Ces constituants ne sont pas représentés.

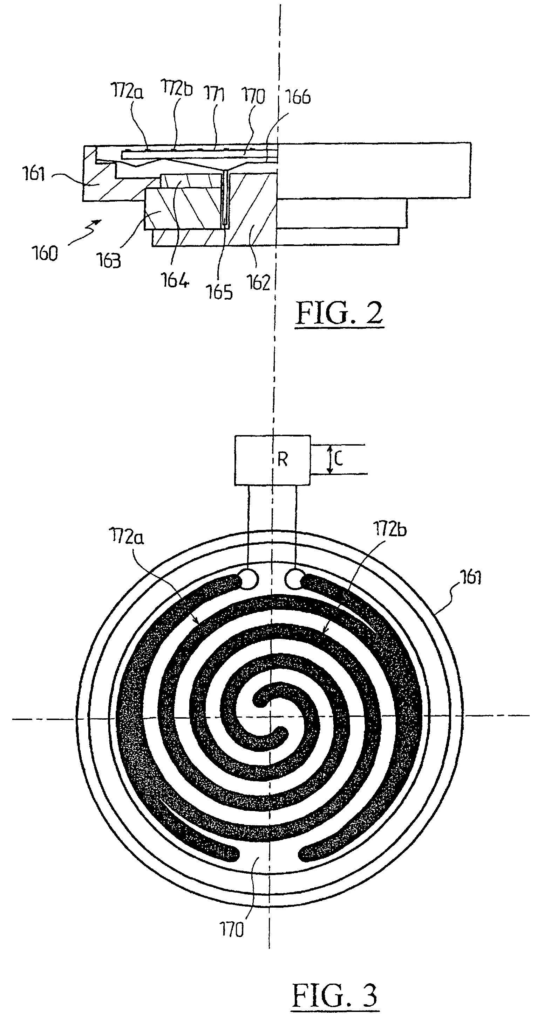

A la Fig. 2, le transducteur électroacoustique 160 est constitué de manière connue d'un logement en matière plastique 161 dans lequel est monté de manière concentrique un moteur magnétique constitué d'une plaque de base en acier 162, d'une bague en matériel magnétique 163, d'un anneau en acier 164, et d'une bobine mobile 165.

Une membrane 166 est fixée, d'une part, à l'intérieur du logement 161 et, d'autre part, à une extrémité de la bobine mobile 165.

Un senseur 170 est collé sur la membrane 166. Il est destiné à transmettre à un doigt de l'utilisateur et en particulier le pouce, les vibrations de la membrane 166. Il intervient également dans la mise sous tension temporaire du dispositif.

Il est constitué d'un support 171 isolant de type circuit imprimé dont la face libre est pourvue de bandes conductrices 172a et 172b en cuivre disposées de manière non sécante et sont isolées électriquement les unes des autres.

Dans un mode de réalisation préféré, les bandes conductrices 172a et 172b forment chacune une spirale, comme cela est visible à la Fig. 3.

Chaque bande conductrice 172a ou 172b est raccordée à un détecteur de contact.

La résistivité de la peau est utilisée pour mettre en oeuvre le détecteur de contact R. Celui-ci ouvre ou ferme un contact électrique C suivant qu'un contact tactile est réalisé ou ne l'est pas sur les bandes conductrices 172a et 172b.

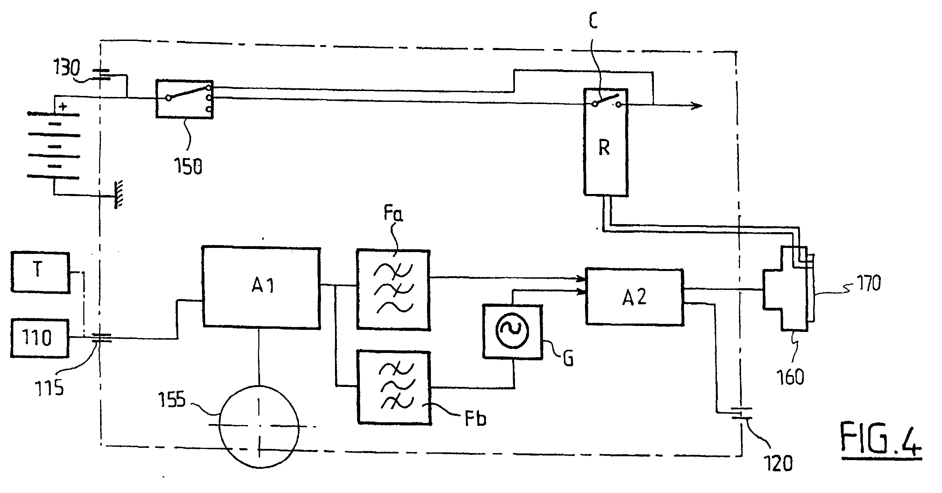

A la Fig. 4, la vue schématique d'un dispositif de stimulation tactile selon l'invention expose ses principales fonctions.

Les composants montés sur le circuit imprimé sont représentés à l'intérieur d'un cadre rectangulaire dessiné en traits mixtes fins.

La voix d'un interlocuteur est captée par le microphone amovible 110 ou par un accessoire de réception T tel qu'un récepteur à boucles magnétiques, monté dans le connecteur électrique d'entrée 115.

Le récepteur à boucles magnétiques utilise un champ magnétique produit par une boucle magnétique.

Les boucles magnétiques sont utilisées dans certaines salles de conférences et permettent la transmission de la voix du conférencier ou d'une traduction de son discours, par couplage magnétique avec des récepteurs dont sont équipés les auditeurs.

Puis le signal produit par le microphone amovible 110 ou par un accessoire de réception T est transmis à un premier amplificateur A1.

Le signal est ensuite transmis en parallèle vers deux filtres fréquentiels Fa et Fb. Le premier filtre Fa passe-bas sélectionne les fréquences jusqu'à 800 Hz, puis le signal filtré est transmis vers une première entrée d'un second amplificateur de puissance A2.

Le second filtre Fb passe-bande sélectionne les fréquences comprises entre 1000 Hz et 4000 Hz, puis fournit le signal filtré vers un générateur de fréquence G qui module en amplitude le signal dans une fréquence de 900 Hz.

Le signal produit par le générateur de fréquence G est ensuite introduit dans une seconde entrée de l'amplificateur A2.

Le signal produit par l'amplificateur A2 est utilisé pour mettre en oeuvre le transducteur électroacoustique 160.

Ainsi, les fréquences aiguës produites par la voix humaine comme les sifflantes "CHE" et présentant un intérêt pour la compréhension de la voix humaine par une personne sourde sont rendues perceptibles par le dispositif de l'invention.

Le signal produit par l'amplificateur A2 est également délivré au connecteur électrique de sortie 120.

D'autres types de transducteurs électroacoustiques peuvent être raccordés au connecteur électrique de sortie 120 comme un transducteur électroacoustique extérieur destiné à être utilisé entre les doigts de la personne.

Le détecteur de contact R raccordé au senseur 170 du transducteur électroacoustique 160 est monté en série avec le bouton de mise sous tension 150 lorsqu'il est commuté en position de veille.

Lorsqu'une personne souhaite utiliser le dispositif de stimulation tactile pour comprendre la voix de son interlocuteur, elle prend dans une main le dispositif, le met éventuellement sous tension ou en position de veille avec le bouton de mise sous tension 150.

Si le dispositif est sous tension, la personne dirige, le cas échéant, le dispositif de manière à ce que le microphone amovible 110 soit orienté en direction du lieu d'où provient la voix.

La personne met en contact le pouce avec le senseur 170 afin de percevoir-et interpréter les vibrations émises par ledit transducteur électroacoustique 160.

Si le dispositif est en position de veille, la mise en contact du pouce de l'utilisateur avec les bandes conductrices 172a et 172b du senseur 170 a pour effet d'induire une résistance électrique non nulle entre les bandes conductrices 172a et 172b. La mesure de cette résistance électrique est prise en compte par le détecteur de contact R qui ferme le contact électrique C et met en marche le dispositif.

La personne peut alors percevoir et interpréter les vibrations émises par ledit transducteur électroacoustique 160.

La molette de réglage 155 peut être utilisée pour permettre de moduler la puissance délivrée par le transducteur électroacoustique 160 afin d'adapter le dispositif aux conditions de perception de la personne qui l'utilise.

Le dispositif de stimulation tactile de l'invention permet de transformer les sons émis par la voix d'une personne en vibrations tactiles pouvant être perçues par la peau d'une personne, en particulier l'extrémité d'un doigt qui est une zone de la peau particulièrement innervée et sensible à la perception spécifique du sens du toucher.

Le dispositif de stimulation tactile de l'invention est d'une construction compacte, ce qui permet son transport dans une poche.

Tous ses composants sont intégrés dans un seul boîtier.

Son utilisation peut démarrer sans aucune préparation pour la perception des voix et de l'ambiance sonore.

Ce dispositif peut ainsi être utilisé immédiatement par une personne sourde lors de situations quotidiennes les plus courantes, comme lorsqu'elle doit répondre à un interphone ou lorsqu'elle doit parler à une personne dans la rue.

Les informations transmises sous forme de vibrations tactiles par le dispositif à la personne qui l'utilise couvrent certaines bandes de fréquences portées par la voix humaine et filtre certaines fréquences parasites.

L'usage du dispositif de stimulation tactile de l'invention peut aussi être envisagé pour l'apprentissage de la compréhension vibrotactile des phonèmes dans la lecture labiale.

Le dispositif de stimulation tactile de l'invention peut être également utilisé par une personne sourde pour lui apprendre à maîtriser sa voix.

Le dispositif de stimulation tactile de l'invention permet également à son utilisateur d'apprécier le niveau de bruit ambiant pour ajuster sa propre voix.

Dans une variante de construction simplifiée, les filtres Fa et Fb, ainsi que le générateur de fréquence G, ne sont pas prévus dans le dispositif de l'invention.

## Revendications

1. Dispositif de stimulation tactile destiné à être utilisé par une personne sourde comportant notamment un transducteur électroacoustique (160), **caractérisé en ce qu'**il est constitué d'un boîtier (100) préhensible dans une main et comportant un senseur (170) de mise sous tension associé au transducteur électroacoustique (160), et destiné à permettre la mise sous tension du dispositif lorsque l'on touche ledit senseur.

2. Dispositif selon la revendication 1, **caractérisé en ce que** le senseur (170) est constitué d'un support (171) isolant pourvu de bandes conductrices (172a, 172b) isolées électriquement les unes des autres et raccordées à l'entrée d'un détecteur de contact (R).

3. Dispositif selon la revendication 2, **caractérisé en ce que** les bandes conductrices (172a, 172b) ont chacune la forme d'une spirale et sont imbriquées les unes dans les autres.

4. Dispositif selon l'une des revendications 1 à 3, **caractérisé en ce que** le senseur (170) est collé sur la membrane (166) du transducteur électroacoustique (160).

5. Dispositif selon l'une des revendications précédentes, **caractérisé en ce qu'**il comporte un filtre fréquentiel (Fa) passe-bas qui sélectionne les fréquences inférieures à 800 Hz.

6. Dispositif selon l'une des revendications précédentes, **caractérisé en ce qu'**il comporte un filtre fréquentiel (Fb) passe-bande raccordé à un générateur de fréquence (G) apte à moduler en amplitude sur une fréquence déterminée le signal reçu dudit filtre fréquentiel.

7. Dispositif selon la revendication 6, **caractérisé en ce que** le filtre fréquentiel (Fb) passe-bande sélectionne la plage de fréquences comprise entre 1000 Hz et 4000 Hz.

8. Dispositif selon la revendication 6 ou 7, **caractérisé en ce que** la fréquence de fonctionnement du générateur de fréquence (G) est de 900 Hz.

9. Dispositif selon l'une des revendications 2, 3 et 4 à 8 quand ces dernières sont basées directement ou indirectement sur la revendication 2 ou 3 comportant un bouton de mise sous tension (150), **caractérisé en ce que** le bouton de mise sous tension (150) peut prendre une position d'arrêt destinée à couper l'alimentation électrique du dispositif, une position de mise sous tension du dispositif et une position de veille pouvant mettre en marche le dispositif par l'intermédiaire du détecteur de contact (R).

10. Dispositif selon l'une des revendications précédentes, **caractérisé en ce qu'**il comporte un connecteur d'entrée (115) prévu pour recevoir un microphone amovible (110) ou un accessoire de réception T comme un récepteur à boucles magnétiques.

11. Dispositif selon l'une des revendications précédentes, **caractérisé en ce qu'**il comporte un connecteur de sortie (120) prévu pour recevoir un transducteur électroacoustique extérieur.

12. Dispositif selon l'une des revendications précédentes, **caractérisé en ce qu'**il comporte une molette de réglage (155) permettant d'agir sur un potentiomètre afin de moduler l'intensité du courant électrique destiné à alimenter le transducteur électroacoustique (160).

## Patentansprüche

1. Vorrichtung zur taktilen Stimulation, die dazu bestimmt ist, von einer tauben Person verwendet zu werden und insbesondere einen elektroakustischen Wandler (160) umfasst, **dadurch gekennzeichnet, daß** sie von einem Gehäuse (100), das in einer Hand gehalten werden kann, gebildet ist und einen Sensor (170) zur Unterspannungsetzung umfasst, der mit dem elektroakustischen Wandler (160) verbunden und dazu bestimmt ist, die Unterspannungsetzung der Vorrichtung zu ermöglichen, wenn der Sensor berührt wird.

2. Vorrichtung nach Anspruch 1, **dadurch gekennzeichnet, daß** der Sensor (170) von einem Isolierträger (171) gebildet ist, der mit leitenden Bändern (172a, 172b) versehen ist, die elektrisch voneinander getrennt und mit dem Eingang eines Kontaktdetektors (R) verbunden sind.

3. Vorrichtung nach Anspruch 2, **dadurch gekennzeichnet, daß** die leitenden Bänder (172a, 172b) jeweils die Form einer Spirale aufweisen und ineinander verschachtelt sind.

4. Vorrichtung nach einem der Ansprüche 1 bis 3, **dadurch gekennzeichnet, daß** der Sensor (170) auf die Membran (166) des elektroakustischen Wandlers (160) geklebt ist.

5. Vorrichtung nach einem der vorhergehenden Ansprüche, **dadurch gekennzeichnet, daß** sie einen Tiefpassfrequenzfilter (Fa) umfasst, der die Frequenzen unter 800 Hz auswählt.

6. Vorrichtung nach einem der vorhergehenden Ansprüche, **dadurch gekennzeichnet, daß** sie einen Bandfrequenzfilter (Fb) umfasst, der mit einem Frequenzgenerator (G) verbunden ist, der das von dem Frequenzfilter empfangene Signal auf einer bestimmten Frequenz amplitudenmodulieren kann.

7. Vorrichtung nach Anspruch 6, **dadurch gekennzeichnet, daß** der Bandfrequenzfilter (Fb) den Frequenzbereich zwischen 1000 Hz und 4000 Hz auswählt.

8. Vorrichtung nach Anspruch 6 oder 7, **dadurch gekennzeichnet, daß** die Betriebsfrequenz des Frequenzgenerators (G) 900 Hz beträgt.

9. Vorrichtung nach einem der Ansprüche 2, 3 und 4 bis 8, wenn diese letztgenannten direkt oder indirekt auf dem Anspruch 2 oder 3 basieren, umfassend einen Knopf (150) zur Unterspannungsetzung, **dadurch gekennzeichnet, daß** der Knopf zur Unterspannungsetzung (150) eine Halteposition, die dazu bestimmt ist, die elektrische Stromversorgung der Vorrichtung zu unterbrechen, eine Position zur Unterspannungsetzung der Vorrichtung und eine Bereitschaftsposition einnehmen kann, die die Vorrichtung über den Kontaktdetektor (R) in Gang setzen kann.

10. Vorrichtung nach einem der vorhergehenden Ansprüche, **dadurch gekennzeichnet, daß** sie einen Eingangsstecker (115) umfasst, der dazu vorgesehen ist, ein abnehmbares Mikrofon (110) oder ein Empfangszubehör T, wie einen Magnetschleifenempfänger, aufzunehmen.

11. Vorrichtung nach einem der vorhergehenden Ansprüche, **dadurch gekennzeichnet, daß** sie einen Ausgangsstecker (120) umfasst, der dazu vorgesehen ist, einen äußeren elektroakustischen Wandler aufzunehmen.

12. Vorrichtung nach einem der vorhergehenden Ansprüche, **dadurch gekennzeichnet, daß** sie ein Einstellrad (155) umfasst, das es ermöglicht, auf ein Potentiometer einzuwirken, um die elektrische Stromstärke, die dazu bestimmt ist, den elektroakustischen Wandler (160) zu speisen, zu modulieren.

## Claims

1. Tactile stimulation device intended for use by a deaf person including in particular an electro-acoustic transducer (160), **characterised in that** it comprises a unit (100) which can be gripped in one hand and includes a power-on sensor (170) linked to the electro-acoustic transducer (160) and intended to enable the device to be switched on when the said sensor is touched.

2. Device according to Claim 1, **characterised in that** the sensor (170) comprises an insulating support (171) fitted with conducting strips (172a, 172b) electrically insulated from one another and connected to the input to a contact detector (R).

3. Device according to Claim 2, **characterised in that** the conducting strips (172a, 172b) each take the form of a spiral and are nested in one another.

4. Device according to one of Claims 1 to 3, **characterised in that** the sensor (170) is glued to the diaphragm (166) of the electro-acoustic transducer (160).

5. Device according to one of the above claims, **characterised in that** it includes a low-pass frequency filter (Fa) which selects frequencies below 800 Hz.

6. Device according to one of the above claims, **characterised in that** it includes a passband frequency filter (Fb) connected to a frequency generator (G) capable of amplitude modulating the signal received from the said frequency filter at a given frequency.

7. Device according to claim 6, **characterised in that** the passband frequency filter (Fb) - selects the range of frequencies between 1000 Hz and 4000 Hz.

8. Device according to claim 6 or 7, **characterised in that** the operating frequency for the frequency generator (G) is 900 Hz.

9. Device according to one of claims 2, 3 and 4 to 8, when the latter are based directly or indirectly on claim 2 or 3, including a power-on button (150), **characterised in that** the power-on button (150) is able to adopt a stop position intended to cut off the electrical power supply to the device, a power-on position for the device and a stand-by position capable of starting the device by means of the contact detector (R).

10. Device according to one of the above claims, **characterised in that** it includes an input connector (115) designed to accept a detachable microphone (110) or a T reception accessory such as a magnetic loop receiver.

11. Device according to one of the above claims, **characterised in that** it includes an output connector (120) designed to accept an external electro-acoustic transducer.

12. Device according to one of the above claims, **characterised in that** it includes an adjustment knob (155) acting on a potentiometer in order to modulate the intensity of the electrical current intended to supply the electro-acoustic transducer.
